# EUROPÄISCHE PATENTSCHRIFT

(11) **EP 0 890 568 B1**
(45) Veröffentlichungstag und Bekanntmachung des Hinweises auf die Patenterteilung: **29.08.2001**
(21) Anmeldenummer: 98111965.4
(22) Anmeldetag: 29.06.1998
(51) Int. Cl.: C07C 67/08, C07C 69/54, C07C 69/52, C07C 67/60, C07C 67/58

(54) **Verfahren zur lösemittelfreien Herstellung von ungesättigten Polyolestern**
Process for the solvent-free preparation of unsaturated polyolesters
Procédé de préparation sans solvant d'esters insaturés de polyols

(30) Priorität: 07.07.1997 DE 19728898
(43) Veröffentlichungstag der Anmeldung: 13.01.1999
(73) Patentinhaber: Cognis Deutschland GmbH, 40589 Düsseldorf (DE)
(72) Erfinder: Le Hen Ferrenbach, Catherine, 77100 Meaux (FR); Guegan, Michel, 77165 Iverny (FR)

(56) Entgegenhaltungen:
- EP-A- 0 376 089
- DE-A- 2 149 530
- DATABASE WPI Week 8650 Derwent Publications Ltd., London, GB; AN 86-328128 XP002079931 & JP 61 243046 A (NIPPON OIL & FATS CO LTD), 29. Oktober 1986

## Beschreibung

Die Erfindung betrifft ein lösemittelfreies Verfahren zur Herstellung von Polyolestem, speziell (Meth)-acrylsäureestern

### Stand der Technik

Ester ungesättigter Carbonsäuren mit Polyolen, wie beispielsweise Acrylsäure- und Methacrylsäureester, finden zunehmende Verwendung als hochreaktive Bestandteile in strahlenhärtenden Systemen. Derartige polyfunktionellen Ester können beispielsweise als Lackrohstoffe für die Elektronenstrahlhärtung oder als Bestandteil von UV-härtenden Druckfarben, Überzugslacken, Spachtel-, Form oder Vergußmassen sowie in Klebstoffen, insbesondere anaerob härtenden Klebstoffen Einsatz finden. Die Anforderungen an die Ester sind indes vielfältig: Neben einer niedrigen Säurezahl und hoher Lagerstabilität müssen sie frei von Eigengeruch sein, praktisch farblos und trübungsfrei. Infolge der hohen Molekulargewichte kommt eine destillative Nachreinigung der Produkte nicht in Frage; auch die Behandlung mit Bleichmitteln ist wenig erfolgreich. Demzufolge besteht besonderes Interesse an Verfahren, die diese Ester unmittelbar in der gewünschten Qualität liefem.

Zur Herstellung der polyfunktionellen Ester existiert eine umfangreiche Vorliteratur. Aus der Deutschen Auslegeschrift **DE-AS 1267547** sowie der Publikation **Chem.lnd. 18, 597 (1970)** sind beispielsweise Verfahren zur Herstellung von polyfunktionellen (Meth)acrylsäureestem bekannt, bei denen man die ungesättigten Carbonsäuren in Gegenwart von sauren Katalysatoren und Polymerisationsinhibitoren wie Phenolen, Phenolderivaten, Cupfer, Cupferverbindungen oder Phenothiazin einer azeotropen Veresterung unterwirft. Die Mitverwendung von Lösemitteln wie Toluol oder Cyclohexan bietet dabei den Vorteil eines leichteren Wärmeübergangs in der Reaktionsmischung und damit kürzeren Veresterungszeiten sowie geringeren Problemen bei der Abtrennung des Kondensationswassers, von Nachteil ist jedoch, daß das Lösemittel nach der Reaktion mit hohem technischen Aufwand abgetrennt werden muß und stets Spuren zurückbleiben, die in aller Regel zu einem unangenehmen Eigengeruch führen. Produkte des Handels weisen dementsprechend Gehalte an nicht abreagierter Acrylsäure von ca. 300 ppm und Lösemittelreste in der Größenordnung von 1000 ppm auf. Abhilfe bietet zwar eine Desodorierung, bei der man die Ester mit Wasserdampf behandelt, dieses Verfahren ist jedoch mit hohem Aufwand an Zeit und Energie verbunden und führt zudem auch nicht zu wirklich lösemittelfreien Produkten.

In der Deutschen Offenlegungsschrift **DE-OS 2913218** wird ein alternatives Herstellverfahren unter Verwendung von Lösemitteln vorgeschlagen, bei dem man die Veresterung in Gegensatz von organischen Estern der phosphorigen Säure und phenolischen Polymerisationsinhibitoren durchführt. Der Inhibitor wird dabei vorzugsweise mit Hilfe eines Trägergases zudosiert.

Aus den Deutschen Offenlegungsschriften **DE-A1 3843843, DE-A1 3843854, DE-A1 3843930** und **DE-A1 3843938** sind Verfahren zur Herstellung von Polyolestem der (Meth)acrylsäure bekannt, die ohne Mitverwendung von Lösemitteln durchgeführt werden. Die Lehren dieser Druckschriften gehen dahin, das bei der Veresterung entstehende Kondensationswasser aus der Gasphase des Reaktionsraumes abzuziehen und die Polymerisationsinhibitoren in Form fein verteilter Flüssigkeitströpfchen in den Reaktionsraum zu bringen, wobei als Inhibitoren vorzugsweise sterisch gehinderte Hydrochinone und Tocopherole eingesetzt werden. In der Deutschen Offenlegungsschrift **DE-A1 4019788** wird weiterhin empfohlen, zur Aktivierung der Inhibitoren während der Veresterung Sauerstoff oder Luft durchzuleiten. Gegenstand der **DE-A1 4430086** ist die Verwendung von hypophosphoriger Säure oder Phosphonsäuren als farbstabilisierende Reduktionsmittel während der Veresterung. Ähnlich wie in den vorgenannten Schriften wird schließlich in der Deutschen Offenlegungsschrift **DE-A1 4140373** vorgeschlagen, die Neutralisation der sauren Veresterungsprodukte mit wasserfreien Basen durchzuführen. Dieses Verfahren erweist sich in der Praxis jedoch als wenig effektiv, weil es leicht zur Vergelung der Produkte kommt.

Demzufolge hat die Aufgabe der vorliegenden Erfindung darin bestanden, ein lösemittelfreies Verfahren zur Herstellung von ungesättigten Polyolestem des geschilderten komplexen Anforderungsprofils zur Verfügung zu stellen, mit dessen Hilfe insbesondere die Vergelung in der Neutralisation zuverlässig verhindert werden kann.

### Beschreibung der Erfindung

Gegenstand der Erfindung ist ein Verfahren zur lösemittelfreien Herstellung von ungesättigten Polyolestern durch
(a) Umsetzung ungesättigter Monocarbonsäuren mit 2 bis 10 Kohlenstoffatomen in Gegenwart von sauren Katalysatoren, Polymerisationsinhibitoren und Reduktionsmitteln unter Durchleiten von Sauerstoff oder Sauerstoff enthaltenden Gasgemischen mit Polyolen,
(b) kontinuierlicher Entfernung des Kondensationswassers aus dem Reaktionsgleichgewicht, sowie
(c) anschließende Neutralisation, Trocknung und Filtration des Veresterungsproduktes,
welches sich dadurch auszeichnet, daß man die Neutralisation mit wäßrigen Basen durchführt und die resultierende wäßrige von der organischen Phase durch Zentrifugieren trennt.

Überraschenderweise wurde gefunden, daß bei Verwendung wäßriger Basen in der Neutralisation die unerwünschte Vergelung zuverlässig vermieden wird. Neutralisation und Phasentrennung können entweder nacheinander oder aber kontinuierlich in einer Zentrifuge durchgeführt werden, wobei sich der Einsatz dieses Bauteils durch kurze Belegzeiten und hohe Trennschärfe auszeichnet. Die Kombination der beiden Verfahrensschritte löst die gestellte Aufgabe in zufriedenstellender Weise.

### Ungesättigte Monocarbonsäuren

Typische Beispiele für ungesättigte Monocarbonsäuren, die als Ausgangsstoffe in Frage kommen, sind Acrylsäure, Methacrylsäure und Crotonsäure sowie deren Mischungen.

### Polyole

Polyole, die im Sinne der Erfindung in als Ausgangsstoffe in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Triethylenglycol, Propylenglycol, Butylenglycol, Hexylenglycol sowie Polyethylenglycole mit einem
- durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Des weiteren können als Ausgangsstoffe Addukte von 1 bis 10, vorzugsweise 2 bis 8 Mol Ethylenoxid und/oder Propylenoxid an die genannten Polyole eingesetzt werden.

### Veresterung

Die Veresterung findet in Abwesenheit organischer Lösungsmittel statt. Um den Verlust an Carbonsäure bei der destillativen Abtrennung des Kondensationswassers auszugleichen, setzt man die Carbonsäuren bezogen auf die Mol-Äquivalente der Polyole üblicherweise im Überschuß, vorzugsweise in einem Verhältnis von 1,1 : 1 bis 1,4 : 1 ein. In einer besonderen Ausführungsform, setzt man die Reaktanten zunächst im stöchiometrischen Verhältnis ein und dosiert den Überschuß dann portionsweise nach. Die Veresterung kann bei Temperaturen im Bereich von 50 bis 150°C durchgeführt werden. Im Hinblick darauf, daß bei zu niedrigen Temperaturen die Reaktionsgeschwindigkeit nicht hoch genug ist und bei zu hohen Temperaturen viele Polymerisationsinhibitoren nicht mehr aktiv sind, hat sich ein Temperaturbereich von 70 bis 100°C bewährt. Weiterhin ist es empfehlenswert, die Veresterung unter vermindertem Druck durchzuführen und hierzu einen Druckgradienten von etwa 400 auf etwa 10 mbar anzulegen, der typischerweise das folgende Profil besitzt: 2 h/400 mbar, 1 h/300 mbar, 0,5 h/200 mbar, 0,5 h/100 mbar sowie 0,5 h/20 mbar und weniger. Der Druckgradient wird dabei der Siedekurve angepaßt, so daß stets Wasser und möglichst wenig Carbonsäure abgetrennt wird. Die Veresterungszeit ist natürlich von den Einsatzverhältnissen und der Temperatur abhängig, typischerweise werden jedoch 2 bis 10 und vorzugsweise 5 bis 8 h benötigt, um zu Produkten zu gelangen, die eine Säurezahl im Bereich von 20 bis 40 aufweisen.

### Saure Katalysatoren

Die Auswahl der sauren Katalysatoren ist an sich nicht kritisch. Typische Beispiele sind Schwefelsäure, Methansulfonsäure, p-Toluolsulfonsäure, Sulfobernsteinsäure oder auch tensidische Katalysatoren wie beispielsweise Dodecylbenzolsulfonsäure oder Alkylschwefelsäurehalbester. Die Einsatzmenge der sauren Katalysatoren kann im Bereich von 1 bis 10, vorzugsweise 0,5 bis 5 Gew.-% - bezogen auf die Einsatzstoffe - liegen. Dabei kann es von Vorteil sein, die Menge an Katalysator ebenfalls portionsweise zuzugeben.

### Polymerisationsinhibitoren

Ein wesentlicher Aspekt bei der Durchführung der Veresterung ist es, die Polymerisation der ungesättigten Carbonsäuren zu verhindern. Hierzu werden Inhibitoren eingesetzt, bei denen es sich im wesentlichen um Chinon- bzw. Hydrochinonverbindungen handelt. Typische Beispiele sind neben Brenzcatechin, Resorcin und Hydrochinon sterisch gehinderte Hydrochinone wie beispielsweise Di-tert.Butylhydrochinon sowie vorzugsweise Methylhydrochinon. Weiterhin können auch Pyrogallol, Tocopherole sowie Cupfer und Cupfersalze eingesetzt werden. Die Inhibitoren werden der Reaktionsmischung üblicherweise in Mengen von 200 bis 10.000 und vorzugsweise 300 bis 2.000 ppm - bezogen auf die Einsatzstoffe - zugesetzt. Werden Hydrochinonverbindungen verwendet, hat es sich weiterhin als vorteilhaft erwiesen, als Co-Inhibitoren Aktivkohlen einzusetzen, wobei deren Einsatzmengen dann bei etwa 10 bis 25 Gew.-% der Inhibitormenge liegen. Zur Aktivierung der Inhibitoren ist es erforderlich, Sauerstoff oder ein Sauerstoff enthaltendes Gasgemisch (z.B. Luft oder O₂/N₂) durch den Reaktionsansatz zu leiten. Typischerweise liegen die Strömungsgeschwindigkeiten dabei im Bereich von 50 bis 200 I O₂/h.

### Reduktionsmittel

Im Hinblick auf eine zufriedenstellende Farbqualität, die auch bei längerer Lagerung erhalten bleibt, hat sich die Mitverwendung von Reduktionsmitteln bewährt. Vorzugsweise werden für diesen Zweck hypophosphorige Säure bzw. deren Alkali- oder Erdalkalisalze eingesetzt. Alternativ hierzu können auch Borhydride verwendet werden, was jedoch in der Handhabung aufwendiger ist. Die Menge an Reduktionsmittel liegt üblicherweise in der Größenordnung von 0,1 bis 3, vorzugsweise 0,5 bis 1 Gew.-% - bezogen auf die Menge der Einsatzstoffe.

### Neutralisation und Phasentrennung

Die rohen Veresterungsprodukte weisen noch eine Säurezahl im Bereich von 20 bis 40 auf. Zur Neutralisation werden als Basen wäßrige Lösungen von Alkali- und/oder Erdalkalicarbonaten eingesetzt. Vorzugsweise kommen wäßrige 20 bis 35 Gew.-%ige Natriumcarbonatlösungen zum Einsatz, wobei man bezogen auf die Säurezahl einen molaren Überschuß von Base im Bereich von 5 bis 50, vorzugsweise 20 bis 30 % entsprechend einem pH-Wert von 7,5 bis 10,5 einstellt. Die Neutralisation kann in einem Schritt durchgeführt werden, es ist jedoch auch möglich, den Ester mehrmals abwechselnd mit Wasser und wäßriger Base zu behandeln und zwischen den Schritten eine grobe Phasentrennung durch Dekantieren durchzuführen. Die endgültige Trennung zwischen der organischen Wertphase und der wäßrigen Phase erfolgt jedoch in üblichen Zentrifugen, vorzugsweise vom Typ der Schneckenaustrag-bzw. Dekantierzentrifugen, wie sie beispielsweise bislang bei der Abwasseraufbereitung eingesetzt werden. Die wäßrige Phase kann anschließend aufgearbeitet und die rückgewonnenen ungesättigten Carbonsäuren in den Prozeß zurückgeführt werden.

### Trocknung und Filtration

Die organische Phase enthält noch eine Restfeuchte (< 1 % H₂O) sowie Salze und feste Rückstände aus der Veresterung, die entfernt werden müssen, da sie ansonsten im Produkt eine unerwünschte Trübung hervorrufen. Die Polyolester werden daher zunächst bei 50 bis 80°C und vermindertem Druck (ca. 10 bis 100 mbar) entwässert, wobei es sich empfiehlt, weiterhin Sauerstoff oder Sauerstoff enthaltende Gasgemische durchzuleiten, um keine Einbußen bei der Farbqualität zu erleiden. Anschließend erfolgt eine Abtrennung der festen Bestandteile in einer konventionellen Filterpresse.

### Gewerbliche Anwendbarkeit

Nach dem erfindungsgemäßen Verfahren werden wasserklare Produkte erhalten, die frei von Lösungsmittelspuren sind und auch ohne Desodorierung einen Gehalt von weniger als 100 ppm freier Carbonsäure aufweisen. Die Produkte sind farbstabil und geruchsfrei und können beispielsweise in Abmischung mit Ringöffnungsprodukten von Epoxiden mit Diolen, vorzugsweise Bisphenol A, als Lackrohstoffe für die Elektronenstrahlhärtung oder als Bestandteil von UV-härtenden Druckfarben, Überzugslacken, Spachtel-, Form oder Vergußmassen sowie in Klebstoffen, insbesondere anaerob härtenden Klebstoffen eingesetzt werden.

### Beispiele

**Beispiel 1**. In einen 30-l-Reaktor wurden 9,6 kg (126 Mol) Acrylsäure 4,7 kg (30 Mol = 90 Mol OH-Gruppen) Glycerin+1PO, 1 kg p-Toluolsulfonsäure, 0,05 kg Methylhydrochinon und 0,3 kg hypophosphorige Säure eingewogen. Die Veresterung wurde unter Durchleiten von Luft (100 l/h) und unter Wasserabscheidung durchgeführt. Bei einer maximalen Reaktionstemperatur von 80°C und einem Vakuumprofil von 2 h/400 mbar, 1 h/300 mbar, 0,5 h/200 mbar, 0,5 h/100 mbar und 0,5 h/10 mbar betrug die Veresterungszeit 5 h. Es wurde ein rohes Veresterungsprodukt mit einer Säurezahl von 20 erhalten. Das Rohprodukt wurde unter Zugabe von 20 Gew.-%iger Natriumcarbonatlösung auf pH = 8,5 eingestellt und 2 h bei Raumtemperatur gerührt. Anschließend wurde das Neutralisat in einer Dekantierzentrifuge innerhalb von 10 min in eine wäßrige und eine organische Phase getrennt; der Restwassergehalt der organischen Phase betrug 0,15 Gew.-%. Das Veresterungsprodukt wurde unter erneutem Durchleiten von Luft bei 70°C und 10 mbar von Wasserspuren befreit und dann über eine Filterpresse gegeben. Es resultierte ein wasserklares Veresterungsprodukt mit einer Gardner-Farbzahl < 1.

**Vergleichsbeispiel V1.** Beispiel 1 wurde wiederholt, bei der Neutralisation wurde jedoch über einen Zeitraum von 2 h eine entsprechende Menge festes Natriumhydroxid eingerührt und das Produkt gleichzeitig bei 70°C und 10 mbar von Wasserspuren befreit. Das Produkt konnte infolge Gelbildung nicht filtriert werden.

**Vergleichsbeispiel V2**. Beispiel 1 wurde wiederholt, die Phasentrennung wurde jedoch nicht in einer Zentrifuge, sondern in einem Dekanter durchgeführt (Dekantierzeit 2 h). Es wurde ein Veresterungsprodukt mit einem Wassergehalt von 0,2 Gew.-% erhalten, welches anschließend getrocknet und filtriert wurde.

## Patentansprüche

1. Verfahren zur lösemittelfreien Herstellung von ungesättigten Polyolestem durch
(a) Umsetzung ungesättigter Monocarbonsäuren mit 2 bis 10 Kohlenstoffatomen in Gegenwart von sauren Katalysatoren, Polymerisationsinhibitoren und Reduktionsmitteln unter Durchleiten von Sauerstoff oder Sauerstoff enthaltenden Gasgemischen mit Polyolen,
(b) kontinuierlicher Entfernung des Kondensationswassers aus dem Reaktionsgleichgewicht, sowie
(c) anschließende Neutralisation, Trocknung und Filtration des Veresterungsproduktes,
**dadurch gekennzeichnet**, daß man die Neutralisation mit wäßrigen Basen durchführt und die resultierende wäßrige von der organischen Phase durch Zentrifugieren trennt.

2. Verfahren nach Anspruch 1, **dadurch gekennzeichnet**, daß man ungesättigte Carbonsäuren einsetzt, die ausgewählt sind aus der Gruppe, die gebildet wird von Acrylsäure, Methacrylsäure, Crotonsäure sowie deren Mischungen.

3. Verfahren nach den Ansprüchen 1 und 2, **dadurch gekennzeichnet,** daß man Polyole einsetzt, die ausgewählt sind aus der Gruppe, die gebildet wird von Glycerin, Alkylenglycolen, technischen Oligoglyceringemischen, Hydroxymethylverbindungen, Alkylglucosiden mit 1 bis 8 Kohlenstoffatomen im Alkylrest, Zuckeralkoholen, Zuckern und Aminozuckern sowie deren Addukten mit Ethylenoxid und/oder Propylenoxid.

4. Verfahren nach den Ansprüchen 1 bis 3, **dadurch gekennzeichnet,** daß man die Carbonsäuren bezogen die Mol-Äquivalente der Polyole im Verhältnis 1,1:1 bis 1,4:1 einsetzt.

5. Verfahren nach den Ansprüchen 1 bis 4, **dadurch gekennzeichnet,** daß man die Veresterung bei Temperaturen im Bereich von 50 bis 150°C durchführt.

6. Verfahren nach den Ansprüchen 1 bis 5, **dadurch gekennzeichnet**, daß man die Veresterung unter vermindertem Druck durchführt.

7. Verfahren nach den Ansprüchen 1 bis 6, **dadurch gekennzeichnet**, daß man als Polymerisationsinhibitor Methylhydrochinon einsetzt.

8. Verfahren nach den Ansprüchen 1 bis 7, **dadurch gekennzeichnet,** daß man als Reduktionsmittel hypophosphorige Säure einsetzt.

9. Verfahren nach den Ansprüchen 1 bis 7, **dadurch gekennzeichnet,** daß man als Basen Alkali- und/oder Erdalkalicarbonatlösungen einsetzt.

10. Verfahren nach den Ansprüchen 1 bis 9, **dadurch gekennzeichnet,** daß man die Trocknung bei vermindertem Druck und unter Durchleiten von Sauerstoff oder Sauerstoff enthaltenden Gasgemischen durchführt.

## Claims

1. A process for the solventless production of unsaturated polyol esters by
(a) reacting unsaturated monocarboxylic acids containing 2 to 10 carbon atoms with polyols in the presence of acidic catalysts, polymerization inhibitors and reducing agents while oxygen or oxygen-containing gas mixtures is/are passed through,
(b) continuously removing the water of condensation from the reaction equilibrium and
(c) subsequently neutralizing, drying and filtering the esterification product,
**characterized in that** the neutralization step is carried out with aqueous bases and the resulting aqueous phase is separated from the organic phase by centrifuging.

2. A process as claimed in claim 1, **characterized in that** unsaturated carboxylic acids selected from the group consisting of acrylic acid, methacrylic acid, crotonic acid and mixtures thereof are used.

3. A process as claimed in claims 1 and 2, **characterized in that** polyols selected from the group consisting of glycerol, alkylene glycols, technical oligoglycerol mixtures, hydroxymethyl compounds, alkyl glucosides containing 1 to 8 carbon atoms in the alkyl group, sugar alcohols, sugars and aminosugars and adducts thereof with ethylene oxide and/or propylene oxide are used.

4. A process as claimed in claims 1 to 3, **characterized in that**, based on the mol-equivalents of the polyols, the carboxylic acids are used in a ratio of 1.1:1. to 1.4:1.

5. A process as claimed in claims 1 to 4, **characterized in that** the esterification is carried out temperatures of 50 to 150°C.

6. A process as claimed in claims 1 to 5, **characterized in that** the esterification is carried out under reduced pressure.

7. A process as claimed in claims 1 to 6, **characterized in that** methyl hydroquinone is used as the polymerization inhibitor.

8. A process as claimed in claims 1 to 7, **characterized in that** hypophosphorous acid is used as the reducing agent.

9. A process as claimed in claims 1 to 7, **characterized in that** alkali metal and/or alkaline earth metal carbonate solutions are used as the bases.

10. A process as claimed in claims 1 to 9, **characterized in that** the drying step is carried out under reduced pressure while oxygen or oxygen-containing gas mixtures is/are passed through.

## Revendications

1. Procédé de préparation sans solvant d'esters de polyols insaturés par
(a) réaction d'acide monocarboxyliques insaturés ayant de 2 à 10 atomes de carbone en présence de catalyseurs acides, d'inhibiteurs de polymérisation et de réducteurs en faisant passer de l'oxygène ou des mélanges gazeux contenant de l'oxygène avec des polyols,
(b) élimination en continu de l'eau de condensation du mélange de l'équilibre reactionnel, ainsi que
(c) ensuite neutralisation, séchage et filtration du produit d'estérification,
**caractérisé en ce qu**'
on conduit la neutralisation avec des bases aqueuses et on sépare par centrifugation la phase aqueuse résultante de la phase organique.

2. Procédé selon la revendication 1,
**caractérisé en ce qu**'
on utilise des acides carboxyliques insaturés qui sont choisis dans le groupe formé par l'acide acrylique, l'acide méthacrylique, l'acide protonique ainsi que leurs mélanges.

3. Procédé selon les revendications 1 et 2.
**caractérisé en ce qu**'
on utilise des polyols qui sont choisis dans le groupe formé par la glycérine, les alkylène glycols, les mélanges industriels d'oligoglycérines, les composés d'hydroxyméthyle, les alkylglycosides ayant de 1 à 8 atomes de carbone dans le radical alkyle, les alcools sacchariques, les sucres et les amino-sucres ainsi que leurs produits d'addition avec l'oxyde d'éthylène ou l'oxyde de propylène.

4. Procédé selon les revendications 1 à 3,
**caractérisé en ce qu**'
on utilise les acides carboxyliques, par rapport aux équivalents molaires des polyols, dans une proportion de 1,1:1 à 1,4:1.

5. Procédé selon les revendications 1 à 4,
**caractérisé en ce qu**'
on conduit l'estérification à des températures allant de 50 à 150°C.

6. Procédé selon les revendications 1 à 5,
**caractérisé en ce qu**'
on conduit l'estérification à pression réduite.

7. Procédé selon les revendications 1 à 6,
**caractérisé en ce qu**'
on utilise comme inhibiteur de polymérisation la méthylhydroquinone.

8. Procédé selon les revendications 1 à 7,
**caractérisé en ce qu**'
on utilise comme réducteur l'acide hypophosphoreux.

9. Procédé selon les revendications 1 à 7,
**caractérisé en ce qu**'
on utilise comme bases des solutions de carbonates de métaux alcalins et/ou de carbonates de métaux alcalino-terreux.

10. Procédé selon les revendications 1 à 9.
**caractérisé en ce qu**'
on conduit le séchage à pression réduite et en faisant passer de l'oxygène ou du mélange gazeux contenant de l'oxygène.
